# EUROPEAN PATENT APPLICATION

(11) **EP 4 311 472 A1**
(43) Date of publication of application: **31.01.2024**
(21) Application number: 23188187.1
(22) Date of filing: 27.07.2023
(51) Int. Cl.: A61B 1/00, A61B 1/05, G02B 5/04, G02B 23/24

(54) **OPTICAL SYSTEM FOR IMPLEMENTATION IN AN ENDOSCOPE AND ENDOSCOPE**

(30) Priority: 28.07.2022 DE 102022119018
(71) Applicant: Karl Storz SE & Co. KG, 78532 Tuttlingen (DE)
(72) Inventor: Hegenbarth, Robin, 78532 Tuttlingen (DE)
(74) Representative: Isarpatent

(57) **Abstract**

Optical system for implementation in an endoscope comprising: a most proximal lens with an optical axis, a proximal portion and a distal portion aligned parallel to the optical axis, an image sensor with an active surface containing an array of light sensitive pixels, and a reflective prism. The system is characterized in that the sensor image is placed orthogonal to the optical axis, with the active surface facing the reflective prism, wherein the proximal portion is smaller than the distal portion, wherein the proximal portion overlaps along a length of the image sensor parallel to the optical axis, wherein the distal portion overlaps with a height of the image sensor perpendicularly to the optical axis, and wherein the image sensor can be positioned at a focal position of the optical system.

## Description

### Technical field of the invention

The present invention relates to optical systems in endoscopes, and in particular to optical systems to be used preferably in the distal end of an endoscope as part of the proximal lens of a video objective.

### Background of the invention

Endoscopes are generally imaging systems with a long, thin shaft that are widely used for the inspection of narrow cavities and conduits. The term endoscope or medical endoscope usually refers to scopes of this nature that are used in the medical industry, such as for surgery or imaging of remote areas of a human or animal body. Endoscopes for use in industrial applications are usually referred to as borescopes. The term endoscope in this disclosure refers to endoscopes designed to be used in medical and/or industrial applications, that is medical endoscopes or borescopes. Depending on the application, the diameters of the distal head of an endoscope typically range from between 4-5 mm to one or more centimeters. As mentioned above, such devices are used most prominently for medical applications, however they also have applications in the testing and maintenance of industrial structures (e.g. pipes, conduits, difficult to reach spaces, etc.) in buildings and industrial facilities.

Endoscopy is one of the cornerstones of modern medical technology. For example, endoscopes are routinely used in laparoscopy, gastroenterology, and other minimally invasive surgical and observational interventions, affording access to human tissues through narrow human tracts, and thereby providing a tool for a thorough inspection of the human body, which can lead to early and accurate diagnoses of diseases and other anomalies without the need for the more invasive surgical methods.

Endoscopes are frequently endowed with multifunctional distal heads, where, besides exploration with illumination and associated camera systems, cleaning elements such as flushing nozzles and other tools such as cutting and cauterizing elements, brushes, and suction nozzles, can also be present. In some endoscopes, the distal head of an endoscope can also be employed with a working channel through which various tools may be introduced into the body to provide the necessary intervention.

One of the most important elements of an endoscope is the objective lens system within the distal head, which collects light from the illuminated scene, e.g. from human tissue within the body, and focuses it, in the case of video endoscopes, onto an image sensor unit, generally comprising one or more electronic image sensors. Image data collected by this sensor unit is transferred to a video processor, often referred to as a camera control unit (CCU), which processes the image signals such that they can be viewed on an external monitor, headset, projection screen, etc., and/or stored for later analysis. The ability to view externally displayed live video assists the operator of the endoscope to perform the inspection or intervention.

One of the directions in which improvements in endoscopy takes place is in the design endoscopes with distal tips of an ever smaller cross sectional diameter, preferably without compromising the quality of the images acquired through their optical systems. This desire to create endoscopes with ever smaller distal diameters affects the ability of the optical system to efficiently focus images on the sensor unit.

Different mechanisms to provide for image focusing within the distal heads of endoscopes have been proposed in the literature. In US 2017/0296042 A1, for instance, an endoscope is described with an observation optical system unit comprising a front-group lens unit on a distal end of the optical system, a rear-group lens unit on the proximal end of the optical system, and a moving lens unit that can be advanced or retracted along a guide frame. Through the movement of the moving lens unit, the optical system unit can focus the incoming light from an object to an image pickup device, which comprises an image sensor.

### Summary of the invention

In endoscopes with very thin shafts and correspondingly desired small cross sectional distal heads, the size of the image sensor might be the limiting factor as to the size of the distal head of the endoscope. The size of the active surface of the image sensor may be comparable or bigger than the target diameter of the video objective of the endoscope. Reducing the size of the image sensor itself, with the corresponding reduction of resolution, due to a reduction in overall pixels on the sensor chip, can lead to a corresponding reduction in image resolution and quality. Furthermore, such a customization of the image sensor itself can result in increased production costs.

It is an object of the present invention to provide an optical system for the proximal lens of an endoscope, preferably for endoscopes with very thin shafts and corresponding distal heads with small cross sectional diameters (typically between 3mm and 4mm), where the image sensor can be placed at the optimal focal distance without having to reduce its size.

The purpose of this invention will be achieved through a device with the features disclosed in claim 1. Preferred embodiments of the invention with advantageous features are given in the dependent claims.

A first aspect of the invention provides an optical system for implementation in an endoscope, comprising a most proximal lens with a proximal portion and a distal portion aligned along an X direction, parallel to an optical axis of the most proximal lens, an image sensor with an active surface containing an array of light sensitive pixels, and a reflective prism. The system is characterized in that the image sensor is placed orthogonal to the optical axis of the most proximal lens, with the active surface facing the reflective prism along a Y direction perpendicular to the X direction. The proximal portion is smaller than the distal portion, wherein the proximal portion of the most proximal lens overlaps along a length of the image sensor along the X axis, wherein the distal portion of the most proximal lens overlaps with a height of the image sensor along a Y axis perpendicular to the X axis, and wherein the image sensor can be positioned at a focal position of the optical system.

A most proximal lens is broadly defined as an optical object whose sides do not have the same size and do not necessarily have the same curvature. A most proximal lens can be a compound lens, with different lenses attached to each other, or a simple lens cut in a way such that it is not mirror-symmetric. In the present invention, the most proximal lens is the one closest to the image sensor in the distal head of the endoscope, which is commonly referred to as the proximal lens group of the endoscope.

Here and in the following, the proximal part or proximal portion of a most proximal lens describes the part or portion of the most proximal lens closest to the person handling the endoscope. Correspondingly, the distal part or distal portion of a lens describes the part or portion of the lens closest to the object, for example, a patient.

The X direction is typically parallel to the optical axis of the distal portion of the most proximal lens. The distal portion and the proximal portion of the most proximal lens do not necessarily have the same optical axis; in some configurations there is, therefore, not a single optical axis for the entire most proximal lens. The X direction will also referred to as the axial direction, the distal direction (if an orientation towards the distal end of the endoscope is implied) or the proximal direction (if an orientation towards the proximal end of the endoscope is implied).

The Y and Z directions are orthogonal directions to the X direction and will both be referred to as the radial direction, unless confusion can arise, in which case they will be distinguished. The Y direction is defined orthogonal to the orientation of the active surface of the image sensor, i.e., the side of the image sensor containing light sensitive pixels, such that the reflective prism mounted upon the image sensor has a larger value along the Y axis than the image sensor. With these conventions, we will understand, for instance, "A is under B" to mean that A has a smaller Y coordinate value than B. Likewise, additional relative positional terms like "below", "on top of" or "over" will acquire a well-defined meaning and they will be used hereafter.

With the previous definitions of directions, the image sensor's active surface being orthogonal to the most proximal lens means that the plane of the image sensor is parallel to the XZ direction. In this configuration, the light rays that cross the most proximal lens can only reach the sensor chips if they are directed thereto by the reflective prism.

Cross-sectional areas perpendicular to the X direction are cuts parallel to the YZ plane. The distal portion and the proximal portion of the most proximal lens typically show an outer surface with curvature. By the cross-sectional area of the distal portion and the proximal portion, cuts well into the bulk of the volumes of the proximal and distal portions are meant, for example at a position where the cut does not intersect with the proximal or distal curved end surface. Cross-sectional areas perpendicular to the X direction will also be referred to as transverse cross-sectional areas. In turn, cross-sectional areas parallel to the X direction (or: surface cuts with an orientation vector pointing in a radial direction) will be referred to as longitudinal cross-sectional areas.

The transverse cross-sectional area of the image sensor overlaps with the transverse cross-sectional area of the proximal portion of the most proximal lens if the image sensor and the proximal portion share Y and Z coordinates. Conversely, the transverse cross-sectional area of the image sensor does not overlap with the transverse cross-sectional area of the proximal portion if the image sensor and the proximal portion do not share Y nor Z coordinates. In other words, the configuration described in the first aspect of the invention implies that if the image sensor would move towards the distal direction, it would fit under the proximal portion (or: it would share X coordinates with the proximal portion but not Y nor Z coordinates) but would contact the distal portion.

One of the main benefits underlying the present invention is the optimization of the space occupied by the image sensor in the optical system installed at the distal end of an endoscope, preferably in endoscopes with very thin shafts, i.e., with distal head diameters of the order of millimeters. This is achieved by first placing the image sensor such that it extends along the direction of the scope (the horizontal direction or the X direction). In this configuration, the active surface of the image sensor does not face the X direction.

A reflective prism is installed in order for the light rays to be diverted from the X direction to the Y direction. This reflective prism will preferably be attached to, abutted to, or simply placed in contact with the image sensor. In order to protect the light sensitive pixels from a direct contact with the reflective prism, the image sensor contains a protective layer, e.g., a cover glass. The image sensor may be attached to a guide, which allows the image sensor to be moved in the X direction, towards the proximal portion of the most proximal lens of the endoscope or away from it. The proximal portion of the most proximal lens is configured to have a transverse cross-sectional area smaller than the transverse cross-sectional area of its distal portion.

This is configured such that the image sensor, in its allowed movement along the X axis does not hit or contact the proximal portion of the most proximal lens, but it eventually can hit or contact the (proximal part of the) distal portion of the most proximal lens. In other words, the image sensor is arranged or is arrangeable in a position which would hit or contact the proximal portion of the most proximal lens if the proximal portion had the same size as the distal portion.

Therefore, by reducing the size of the proximal portion of the most proximal lens with respect to the distal portion of the most proximal lens, the image sensor can move under the proximal portion of the most proximal lens towards the distal end of the endoscope. This wider reach in the available positioning of the image sensor guarantees that an optimal focal position for the image sensor can be achieved.

One advantage of the present invention is that one can reduce the size of the video objectives in endoscopes while still using a standard image sensor. This is particularly useful in endoscopes that require a very thin shaft (e.g., in cystoscopes for the urinary tract). This benefit is also useful in general for endoscopes that have complex distal heads with many functionalities, where freed space, as a result of the smaller objective optical systems, can be used for other purposes, such as providing an opening of a working channel or light sources. In these cases, reducing the size of the video objectives without compromising the quality of the images is clearly a desired feature.

Advantageous embodiments and further developments follow from the dependent claims as well as from the description of the different preferred embodiments illustrated in the accompanying figures.

According to some of the embodiments, the most proximal lens is a simple lens (or: monolithic lens). In this case, the proximal portion is defined by a cut-out or recessed portion of this monolithic lens. Knowing the required focal positions of optical system in relation to the image sensor, one can determine how far this cut-out region should extend towards the distal direction of the most proximal lens.

According to some of the embodiments, the most proximal lens comprises a compound lens with two or more lenses attached to each other, usually with an optical cement. The two or more lenses have a common refractive index or different refractive indices. The lens can therefore have a common optical axis or different optical axes depending on physical geometry of the lens. The different combinations of optical properties are to be adjusted such that the light rays coming from the distal head of the endoscope can be focused on the image sensor.

According to some of the embodiments, the distal portion of the most proximal lens has a convex outer surface and/or the proximal portion of the most proximal lens has a concave or flat outer surface. As mentioned above, in all these embodiments the most proximal lens is arranged such that the light rays coming from an object space into the endoscope are focused onto the image sensor.

According to some of the embodiments, the proximal portion of the most proximal lens comprises an indentation. An indentation here refers to any recess, notch or cut that the proximal portion of the most proximal lens shows and reduces its longitudinal cross-sectional area compared to the longitudinal cross-sectional area of distal portion of the most proximal lens. In some applications, this indentation can be a carved-out portion of the most proximal lens on its proximal side. Such a carving could be e.g., a cut parallel to the XZ plane on the proximal side of the most proximal lens from a certain Y value down to the bottom of the most proximal lens.

According to some of the embodiments, the proximal portion of the most proximal lens and the distal portion of the most proximal lens have different, parallel optical axes. The optical axis of the proximal portion of the most proximal lens may preferably be arranged further away from the image sensor than the optical axis of the distal portion, thereby allocating more room for the image sensor to be moved along the distal direction. By displacing the optical axes of the proximal and distal portions from each other, the perpendicular cross-sectional area of the proximal portion can be larger than if the axes were aligned.

This configuration may be particularly advantageous if the most proximal lens comprises two different circular lenses attached to each other, with the proximal lens having a diameter which in a configuration where the proximal portion and the distal portion had a common optical axis would be too big to grant space to the image sensor.

According to some of the embodiments, the reflective prism is a triangular prism reflector. Such a prism receives the light entering the endoscope through the distal head and traversing the most proximal lens and directs it by reflection at a right angle, i.e., the light rays are diverted from the X direction to the Y direction, such that the light can be detected at the image sensor.

According to some of the embodiments, the diameter of the distal portion of the most proximal lens is between 1 mm and 10 mm, preferably between 2.5 mm and 4 mm. The latter diameters define very thin scopes for video objectives in endoscopes.

According to some of the embodiments, the proximal surface of the most proximal lens is concave.

According to some of the embodiments, the image sensor comprises a cover glass positioned between the active surface and the reflective prism.

According to some of the embodiments, the reflective prism is directly adhered to the cover glass.

According to some of the embodiments, the position of the image sensor may be adjusted along an axis parallel to the optical axis.

According to some of the embodiments, the invention also provides an endoscope containing the optical system of the invention.

According to some of the embodiments, an endoscopic system comprising an endoscope containing the optical system of the invention further comprising an image processor is provided.

According to some of the embodiments, an endoscopic system comprising an endoscope containing the optical system of the invention further comprising an illumination source is provided.

When the optical system of the invention is embedded in an endoscope, in some embodiments the image sensor is configured to send a signal to an external video-processing device. The processed images, which can be adjusted to enhance the contrast, exposure, color, etc., can be further displayed in real time or quasi-real time on a screen. In this way a correct differentiation of e.g., human tissues can be aided.

The above embodiments and implementations can be combined with each other as desired, in order to solve specific technical challenges.

Further applicability of the present optical system will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

### Brief description of the figures

Aspects of the present disclosure will be better understood with reference to the following figures. The components in the drawings are not necessarily to scale, emphasis being placed instead upon clearly illustrating the principles of the present disclosure. Parts in the different figures that correspond to the same elements have been indicated with the same reference numerals in the figures, in which:
Fig. 1 is a schematic depiction of the parts and elements of an endoscopic system;
Fig. 2 is a schematic longitudinal cross-sectional view of an optical system according to an embodiment of the present invention, including a most proximal lens, a reflective prism, and an image sensor;
Fig. 3 is a schematic illustration of the longitudinal and transverse cross sections of the most proximal lens of an optical system according to an embodiment of the present invention, where the distal and proximal portions of the most proximal lens have different optical axes;
Fig. 4 is a schematic illustration of the longitudinal and transverse cross sections of the most proximal lens of an optical system according to an embodiment of the present invention, where the proximal portion of the most proximal lens shows a cut-out region defined below a certain value of Y and extending along the Z direction; and
Fig. 5 is a schematic illustration of the longitudinal and transverse cross sections of the most proximal lens of an optical system according to an embodiment of the present invention where the proximal portion shows a box-shaped recess, defined below a certain value of Y and for two limiting values of Z.

The figures are not necessarily to scale, and certain components can be shown in generalized or schematic form in the interest of clarity and conciseness. In some instances, well-known structures and devices are shown in block diagram form in order to avoid obscuring the concepts of the present invention.

### Detailed description of the figures

The detailed description includes specific details for the purpose of providing a thorough understanding of the present invention. However, it will be apparent to those skilled in the art that the present invention may be practiced without these specific details.

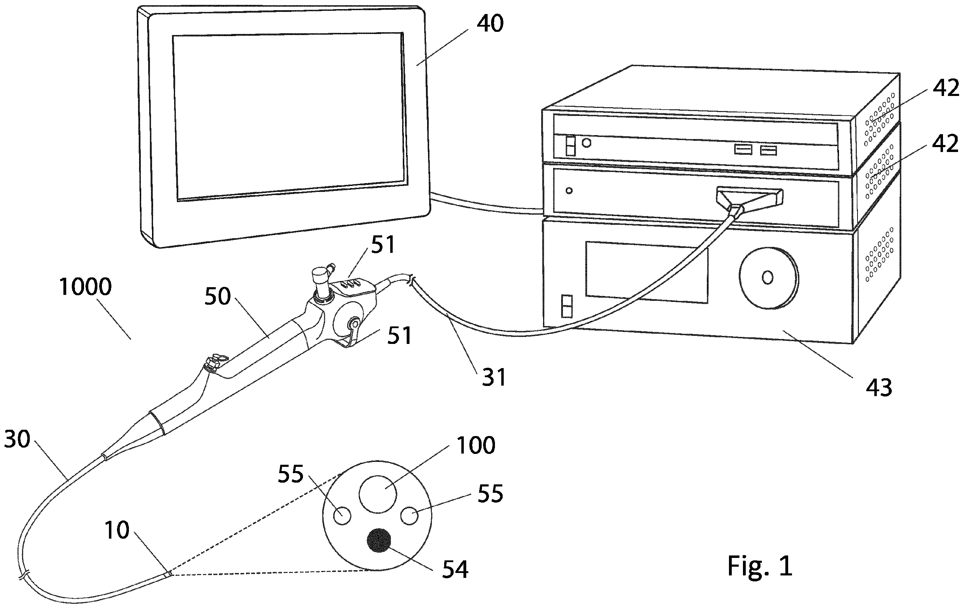

Fig. 1 shows a schematic depiction of the parts and elements that configure an endoscope 1000 according to an embodiment of the present invention. The endoscope 1000 comprises a distal head 10, a shaft 30, a handle 50 and a cable 31. Fig. 1 also shows a display monitor 40 (which may take the form of a standard video display, a projection display, a headset, etc.), a video-processing device (video processor, or CCU) 42 and an illumination source 43.

The distal head 10 is a multifunctional component of the endoscope 1000 that may comprise a plurality of sensors and tools for inspection and intervention. In Fig. 1, the distal head 10 shows, for the purposes of illustration, two light emitters 55, a nozzle 54 for providing a flushing fluid and/or suction, and an optical system 100. The light emitters 55 may by light-emitting diodes (LEDs) installed on the distal head 10 or be the end of a waveguide through which light can be transmitted from an illumination source 43 to the distal head 10. The most distal element of the optical system 100 depicted in Fig. 1 may typically be an objective lens or optical system cover glass. In some embodiments, the most distal element of the optical system acts as both a cover glass and an objective lens. In Fig. 1, the optical systems 100 have different diameters and can be used for different inspection purposes. A distal head 10 can typically be a few centimeters long and can have a transverse cross-sectional diameter of a few millimeters. Some implementations of the distal head may include multiple optical systems; for example, two, independent optical systems may be employed for 3D imaging, or one optical system may be dedicated to white light imaging, while another is dedicated to fluorescent imaging.

The shaft 30 can range in length between approximately 10 cm to a meter long or longer depending on the intended use of the endoscope 1000. The shaft 30 may be rigid or flexible. Within the shaft 30 there may be contained a plurality of communication and/or transport means, e.g., electronic cables to transfer electric signals and power to and from a camera control unit, and/or tubes to carry fluid from an external source to a nozzle 54. Additionally, the shaft 30 may contain a waveguide, e.g., made of optical fiber, to bring light from an external illumination source 43 to the light emitters 55.

The shaft 30 connects the distal head 10 with the rest the handle 50. The shaft 30 may additionally contain, in the region adjacent to the distal head 10, a mechanism (not shown) that allows the distal head 10 to be articulated. This articulation mechanism eases the maneuvering of the distal head 10 to its desired location and permits a visual change in orientation relative to the shaft 30.

The handle 50 is typically a rigid, e.g., a hard-plastic or metallic ergonomic structure, from where the different functions of the endoscope 1000 and/or endoscopic system can be controlled. It typically contains a number of buttons 51 and/or other control mechanisms. The maneuvering of the orientation of the distal head 10, the activation of the nozzle 54, control of the illumination system, and/or the focal adjustment of the optical system 100, to give only some examples, can be performed with the handle 50 through its controls 51.

The video processor 42 is an image-processing unit that receives image data acquired by the optical system 100 and transfer it to a display monitor 40. The video processor 42 may also perform various image processing procedures, such as image contrast and color tonality adjustment. This image-enhancement may provide enhanced differentiation of the tissues under inspection.

The cable 31 is a regular insulated cable for electrical communication between the endoscope handle 50 and the video processor 42. Optionally (not shown in the figure), cable 31 can also be connected to the illumination source 43. In some embodiments it may also be convenient to have the screen 40 and the video processor 42 and/or the illumination source 43 be contained in a single unit.

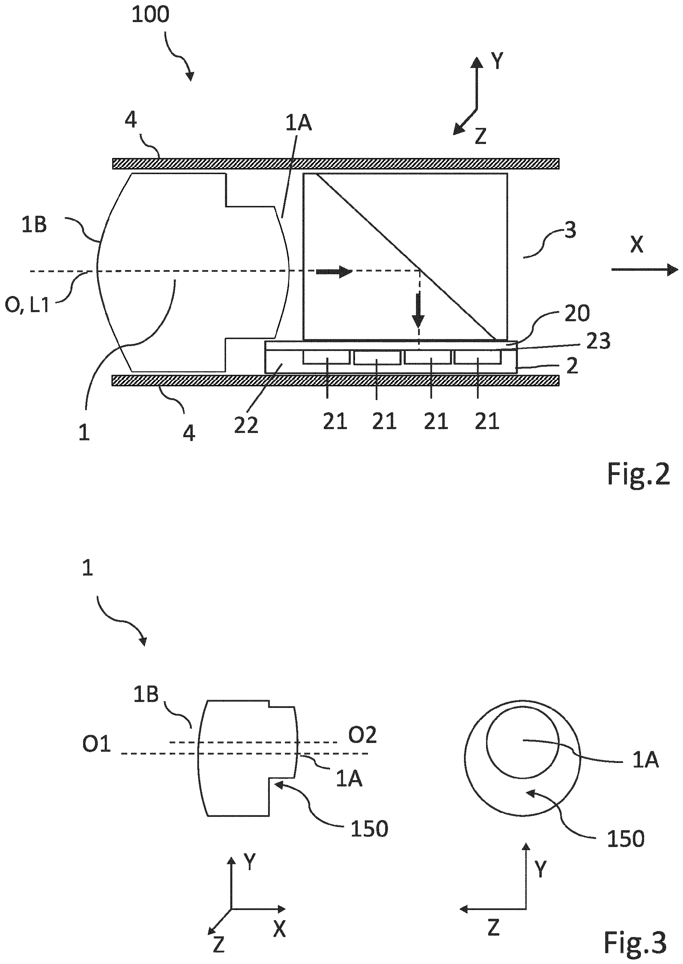

Fig. 2 shows a schematic longitudinal cross-sectional view of the proximal part of an optical system 100 according to an embodiment of the present invention. The optical system 100 in this particular embodiment comprises a most proximal lens 1, an image sensor2, a reflective prism 3, and a holding frame 4. These are the minimal elements relevant to describe an embodiment of the present invention.

However, it should be noted that this simplified optical system 100 can be more sophisticated, in particular comprising further lenses and/or lens groups towards the distal end of the endoscope 1000. In this case, the embodiment depicted in Fig. 2 should be understood as describing a configuration of the proximal lens group of an overall optical system 100.

The most proximal lens 1 has a proximal portion 1A and a distal portion 1B. In the embodiment depicted in Fig. 2, both portions have convex outer surfaces. The diameter of the distal portion 1B is bigger than the diameter of the proximal portion 1A. Thus, the transverse cross section of the distal portion 1B has a larger area than the transverse cross-sectional area of the proximal portion 1A. In Fig. 2, the most proximal lens 1 is depicted as monolithic. In practice, the most proximal lens 1 can be a simple lens or a compound lens, depending on the desired optical properties of the most proximal lens 1.

The image sensor 2 is an image-acquiring structure which usually comprises a protective surface such as a cover glass 20 and an array of light sensitive pixels 21. Commonly used image sensors include CMOS or CCD sensors.

The optical system 100 of Fig. 2 shows a cross section of the image sensor 2 including several representative light sensitive pixels 21 of the overall pixel array. The image sensor 2, which may be mounted on a printed circuit board, is connected by a plurality of leads (not shown in the figure) through an electrical connection along the shaft 30, to the handle 50 and ultimately to the video processor 42. In certain embodiments, some, or all video processing may be performed in the handle 50 itself.

The reflective prism 3 is an optical device configured to reflect the light that impinges on it coming from the most proximal lens and divert it to the image sensor 2. Fig. 2 shows a triangular prism reflector as an example of a reflective prism 3. According to the embodiment of Fig. 2, a light ray L1 coming from an object from outside the endoscope will enter the optical system 100 through the distal portion 1B of the most proximal lens 1.

For simplicity, the light ray L1 under consideration travels along the optical axis of the most proximal lens 1. The light ray L1 therefore traverses the most proximal lens 1 following a straight trajectory and exits the most proximal lens 1 at the proximal portion 1A. The light ray L1 then impinges on the reflective prism 3 and its trajectory is diverted towards the image sensor 2, where the light ray L1 is detected.

In the embodiment shown in Fig. 2, the image sensor 2 is placed such that the plane of its active surface 23, which contains light sensitive pixels 21, faces the Y direction as depicted in Fig. 2. The X direction (or: axial direction) is also shown in Fig. 2, and it is typically parallel to the optical axis O of the most proximal lens 1. In Fig. 2, the distal portion 1B and the proximal portion 1A of the most proximal lens 1 have the same optical axis O, however, in other embodiments this is not necessarily the case.

The Y and Z directions are orthogonal to the X direction. As explained above, the Y direction is orthogonal to the plane of the active surface 23 of the image sensor 2, such that the reflective prism 3 is located at a position farther along the Y axis than is the image sensor 2. The Z direction is therefore defined as orthogonal to the X and Y axes in a Cartesian reference frame.

The placement of the image sensor 2 as shown in the embodiment depicted in Fig. 2, that is parallel to the X axis, is advantageous for those optical systems 100 where the diagonal length of the image sensor 2 is equal or bigger than the maximum diameter of the most proximal lens 1. This is the situation in the embodiment of Fig. 2, where the length of the image sensor 2 (i.e., the size of the image sensor 2 along the X direction) is bigger than the diameter of the holding frame 4, usually defined by the biggest diameter of the most proximal lens 1. The diameter of the holding frame 4 may also define the diameter of the distal head, and thus the shaft 30.

With the arrangement of the image sensor 2 depicted in Fig. 2, the reflective prism 3 is arranged such that light rays, such as light ray L1, reach the image sensor2. In Fig. 2 the reflective prism 3 is placed in contact with the cover glass 20 of the image sensor 2.

The holding frame 4 covers and protects the optical components within the optical system 100. Its inner diameter is typically set by the size of the most proximal lens 1. In order to distinguish both elements more clearly, a small separation is shown between the holding frame 4 and the most proximal lens 1 in Fig. 2.

The image sensor 2, together with the reflective prism 3, may be moved along the X direction, e.g., with the help of a guide rail (not shown in the figure), such that the image sensor 2 can be placed at an optimal focal position. In order to reach an optimal focal position with configurations such as the one depicted in Fig. 2, the image sensor 2 is required to be rather close to the distal portion 1B of the most proximal lens 1. In order to accommodate the positioning of the lens 1 relative to the image sensor 2, the transverse cross-section of the proximal portion 1A of the most proximal lens 1 is reduced is to grant space for the image sensor 2 to get closer to the distal portion 1B without the peripheral edge of the most proximal lens 1 coming into contact with the image sensor 2 and/or its cover glass 20. Fig. 2 shows such a configuration wherein the image sensor 2 has a protruding part 22 that is able to be shifted under the proximal portion 1A of the image sensor 2. This allows the reflective prism to be placed in an optimal position in order to direct an in-focus image onto the image sensor. Thus, the protruding part 22 of the image sensor 2 is located, at least in part, under the proximal portion 1A if the image sensor 2.

Fig. 3 shows a schematic illustration of the longitudinal and transverse cross sections of the most proximal lens 1 of an optical system 100 according to another embodiment of the present invention, where the distal portion 1B and the proximal portion 1A have different, though parallel, optical axes O1, O2. The illustration on the left-hand side of the figure shows a longitudinal cross-sectional view of a most proximal lens 1 along the XY plane.

In this illustration, the most proximal lens 1 consists of a circular cross-sectional proximal portion 1A and a circular cross-sectional distal portion 1B, where the diameter of the proximal portion 1A is smaller than the diameter of the distal portion 1B because of the presence of a recess 150, which lies on a plane parallel to the YZ plane. In contrast to the embodiment of the most proximal lens 1 shown in Fig. 2, in Fig. 3 the optical axis O2 of the proximal portion 1A is arranged further away from the image sensor 2 (not shown in the figure) than the optical axis O1 of the distal portion 1B. In other words, the optical axis O2 of the proximal portion 1A has a higher value along the Y axis than the optical axis O1 of the distal portion 1B.

The illustration on the right-hand side of Fig. 3 shows a transverse cross-sectional view of the corresponding most proximal lens 1 along a plane perpendicular to the X direction, seen from the proximal side of the most proximal lens 1. In this transverse cross section, the shape of the recess 150 is more clearly illustrated as an annular region constrained between two circumferences, the inner circumference being centered at a higher Y coordinate than the outer circumference.

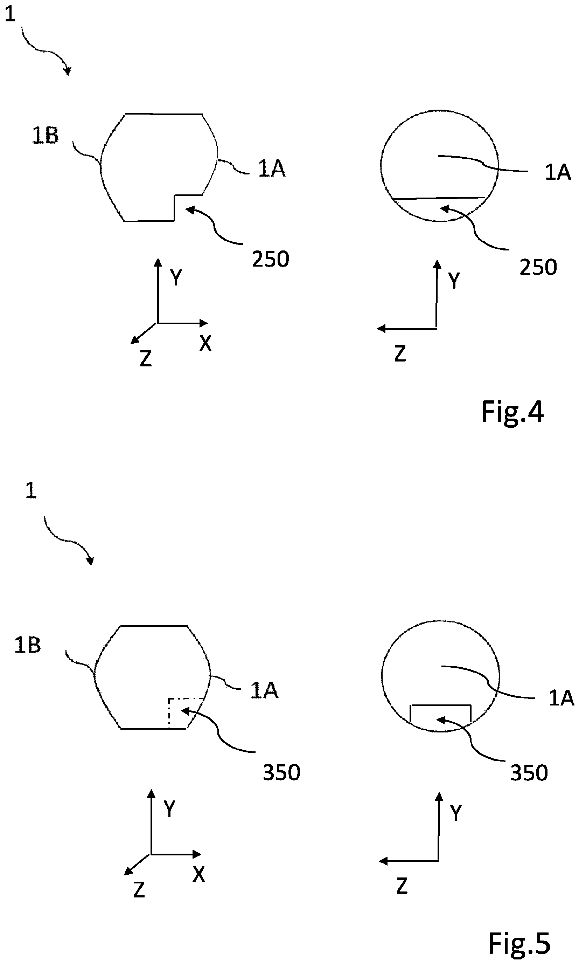

Fig. 4 shows a schematic illustration of the longitudinal and transverse cross sections of the most proximal lens 1 of an optical system 100 according to an alternate embodiment of the present invention, where the proximal portion 1A contains a cut-out region or recess 250 along a plane parallel to the YZ plane defined at a fixed X coordinate, below a certain value of Y and extending along the Z direction.

The illustration on the left-hand side of Fig. 4 shows a longitudinal cross-sectional view of a most proximal lens 1 along the XY plane. In this illustration, the most proximal lens 1 consists of a circular cross sectional distal portion 1B and a proximal portion 1A, where the proximal portion 1A contains a recess 250. In the embodiment of Fig. 4, the proximal portion 1A has the same curvature as the distal portion 1B, such that without the presence of the recess 250 the two sides of the lens would be mirror-symmetric with respect to the X direction; however, this symmetry is only present for illustrative purposes. In practice, the radii of curvature for the proximal portion 1A and distal portion 1B might be distinctly different. Further, one face of the most proximal lens might be convex while the other face is concave.

The illustration on the right-hand side of Fig. 4 shows a transverse cross-sectional view of the corresponding most proximal lens 1 along a plane perpendicular to the X direction, seen from the proximal side of the most proximal lens 1. In this transverse cross section, the shape of the recess 250 is more clearly illustrated as a truncated disk region, defined by a cut-out section at a maximal value of Y along the full extent of the transverse cross section of the most proximal lens 1.

In Fig. 4, both the height and depth of the recess 250, are values that are adjusted based on the space requirements of the image sensor 2, in order to accommodate the positioning of the protruding part 22 of the image sensor 2 (see e.g., Fig. 2) beneath the proximal portion 1A of the most proximal lens 1.

Fig. 5 shows a schematic illustration of the longitudinal and transverse cross sections of the most proximal lens 1 of an optical system 100 according to another embodiment of the present invention, where the proximal portion 1A shows a cut-out region 350 defined at a fixed value of X below a certain maximal value of Y, and for two limiting values of Z that lie within the transverse cross section of the most proximal lens 1. This cut-out region 350 describes the end surface of a box-shape recess on the proximal side of the most proximal lens 1.

The illustration on the left-hand side of Fig. 5 shows a longitudinal cross-sectional view of a most proximal lens 1 along the XY plane. In this illustration, the most proximal lens 1 consists of a circular cross sectional distal portion 1B and a proximal portion 1A, where the proximal portion 1A contains the recess 350. As shown, only for the sake of clarity, the embodiment of Fig. 5 shows the proximal portion 1A as having the same curvature as the distal portion 1B, such that without the presence of the recess 350 the two sides of the lens would be mirror-symmetric with respect to the X direction. Again, as with all other embodiments disclosed herein, there is no requirement that the most proximal lens 1 have equal radii of curvature on their entrance and exit surfaces. Dashed lines indicate the recessed region, while elements, better seen in the illustration on the right of Fig. 5, may be present along the Z axis.

The illustration on the right-hand side of Fig. 5 shows a transverse cross-sectional view of the corresponding most proximal lens 1 along a plane perpendicular to the X direction, seen from the proximal side of the most proximal lens 1. In this transverse cross section, the shape of the recess 350 is better illustrated as a step-shaped region, defined below a maximal value of Y and along the transverse cross section of the most proximal lens 1 between two values of Z which lie within the transverse cross section of the most proximal lens 1.

In Fig. 5, the height, length and depth of the recess 350 values that are selected and/or adjusted based on the space requirements of the image sensor 2, in order to accommodate the positioning of the protruding part 22 of the image sensor 2 (see e.g., Fig. 2) beneath the proximal portion 1A of the most proximal lens 1.

In all embodiments described in Figs. 2, 3, 4 and 5, the proximal portion 1A of the most proximal lens 1 shows a convex outer surface. However, as discussed above, additional embodiments where the outer surface of the proximal portion 1A and/or the distal portion 1B of the most proximal lens 1 are concave or flat, or have a variable radius of curvature, such as with an aspherical lens, are possible.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure.

Thus, the present invention is not intended to be limited to the embodiments shown herein but it is to be accorded the widest scope consistent with the principles and novel features disclosed herein. Therefore, the present invention is not to be limited except in accordance with the following claims.

## Claims

1. An optical system (100) for implementation in an endoscope comprising:
a most proximal lens (1) with an optical axis (O), a proximal portion (1A) and a distal portion (1B) aligned parallel to the optical axis (O);
an image sensor (2) with an active surface (23) containing an array of light sensitive pixels (21); and
a reflective prism (3);
**characterized in that** the image sensor (2) is placed orthogonal to the optical axis (O), with the active surface (23) facing the reflective prism (3), wherein the proximal portion (1A) is smaller than the distal portion (1B), wherein the proximal portion (1A) overlaps along a length of the image sensor (2) parallel to the optical axis (O), wherein the distal portion (1B) overlaps with a height of the image sensor (2) perpendicularly to the optical axis (O), and wherein the image sensor (2) can be positioned at a focal position of the optical system (100) .

2. The optical system (100) according to claim 1, wherein the most proximal lens (1) is a simple lens.

3. The optical system (100) according to claim 1, wherein the most proximal lens (1) comprises a compound lens.

4. The optical system (100) according to any of the previous claims, wherein the distal portion (1B) of the most proximal lens (1) has a convex outer surface and/or the proximal portion (1A) of the most proximal lens (1) has a concave or flat outer surface.

5. The optical system (100) according to any of the previous claims, wherein the proximal portion (1A) of the most proximal lens (1) and the distal portion (1B) of the most proximal lens (1) have different, parallel optical axes.

6. The optical system (100) according to any of the previous claims, wherein the proximal portion (1A) of the most proximal lens (1) comprises an indentation.

7. The optical system (100) according to any of the previous claims, wherein the reflective prism (3) is a triangular prism reflector.

8. The optical system (100) according to any of the previous claims, wherein the diameter of the distal portion (1B) of the most proximal lens (1) is between 1 mm and 10 mm, preferably between 2.5 mm and 4 mm.

9. The optical system (100) according to any of the previous claims, wherein a proximal surface of the most proximal lens (1) is concave.

10. The optical system (100) according to any of the previous claims, wherein the image sensor (2) comprises a cover glass (20) positioned between the active surface (23) and the reflective prism (3).

11. The optical system (100) according to claim 10, wherein the reflective prism (3) is directly adhered to the cover glass (20).

12. The optical system (100) according to any of the previous claims, wherein the position of the image sensor (2) may be adjusted along an axis parallel to the optical axis (O).

13. An endoscope (1000) comprising the optical system (100) according to any of the previous claims.

14. An endoscopic system comprising the endoscope (1000) of claim 13, further comprising an image processor (42).

15. An endoscopic system according to claim 14, further comprising an illumination source (43).
